# FASCICULE DE BREVET EUROPEEN

(11) **EP 0 610 134 B1**
(45) Date de publication et mention de la délivrance du brevet: **05.04.2000**
(21) Numéro de dépôt: 94400215.3
(22) Date de dépôt: 02.02.1994
(51) Int. Cl.: C07D 403/06, A61K 31/33, C07D 403/14, C07D 413/14, C07D 419/14

(54) **Dérivés de l'indole comme 5-Ht1-like agonistes**
Indolderivate als 5-HT1-like Agonisten
Indole derivatives as 5-HT1-like agonists

(30) Priorité: 02.02.1993 FR 9301054
(43) Date de publication de la demande: 10.08.1994
(73) Titulaire: ADIR ET COMPAGNIE, 92415 Courbevoie Cédex (FR)
(72) Inventeur: Lavielle, Gilbert, F-78170 La Celle Saint Cloud (FR); Maillos, Philippe, F-94800 Villejuif (FR); Muller, Olivier, F-95800 Emmery (FR); Laubie, Michel, F-92420 Vaucresson (FR); Verbeuren, Tony, F-78540 Vernouillet (FR); Descombes, Jean-Jacques, F-93360 Neuilly-Plaisance (FR)

(56) Documents cités:
- EP-A- 0 382 570
- EP-A- 0 494 774
- EP-A- 0 497 512
- WO-A-91/18897
- WO-A-92/06973
- WO-A-93/00333
- DE-A- 3 131 728

## Description

La présente invention concerne de nouveaux dérivés de l'indole, leur procédé de préparation et les compositions pharmaceutiques qui les contiennent.

De nombreux dérivés de l'indole ont été décrits dans la littérature. Certains d'entre eux ont été développés en tant qu'agonistes des récepteurs 5-HT₁₋ₗᵢₖₑ pour le traitement et la prévention des douleurs occasionnées par un flux vasculaire anormal comme la migraine et les maladies associées. C'est le cas plus particulièrement des composés décrits dans EP-A-382570, DE-A-3131728, EP-A-438230, EP-A-486666, WO-A-9206973, EP-A-494774, EP-A-497512, WO-A-9118897 et WO-A-9300333. EP-A-135781 décrit, quant à lui, des dérivés de l'indazole en tant qu'analgésiques centraux ayant des propriétés neuroleptiques.

Les composés de la présente invention, outre le fait qu'ils soient nouveaux, présentent des propriétés pharmacologiques particulièrement intenses.

Plus spécifiquement, la présente invention concerne des composés de formule (I) : 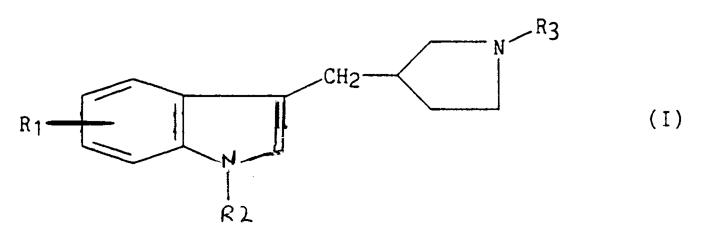 dans laquelle :
- R₁: représente un atome d'hydrogène, d'halogène, un groupement alkyle (C₁-C₆) linéaire ou ramifié, alkoxy (C₁-C₆) linéaire ou ramifié, cyano, aminocarbonyle, ou l'un quelconque des groupements suivants : 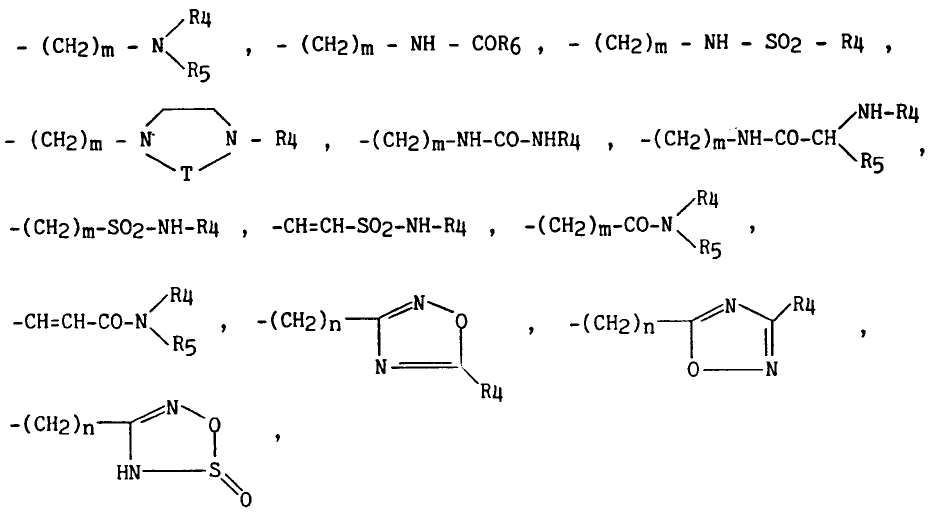 dans lesquels :
- m: est égal à 1, 2 ou 3,
- n: est égal à 0, 1 ou 2,
- T: représente un CO ou SO₂,
- R₄: ou R₅, identiques ou différents, représentent un atome d'hydrogène, un groupement alkyle (C₁-C₆) linéaire ou ramifié ou phényle (substitué ou non par un ou plusieurs atomes d'halogène ou groupements, trihalogénométhyle),
- R₂: représente un atome d'hydrogène, un groupement alkyle (C₁-C₆) linéaire ou ramifie ou phényle (substitué ou non par un ou plusieurs atomes d'halogène ou groupements trihalogénométhyle),
- R₃: représente un atome d'hydrogène, un groupement alkyle (C₁-C₆) linéaire ou ramifié (éventuellement substitué par un groupement hydroxy ou phényle (lui-même substitué ou non par un ou plusieurs atomes d'halogène ou groupements trihalogénométhyle)) ou alkoxycarbonyle (C₁-C₆) linéaire ou ramifié,
leurs énantiomères, diastéréoisomères et épimères ainsi que leurs sels d'addition à un acide pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthane sulfonique, camphorique, oxalique.

La présente invention concerne également le procédé de synthèse des composés de formule (I).

Le procédé de préparation des composés de formule (I) est caractérisé en ce que l'on utilise comme produit de départ une isatine de formule (II) obtenue selon les procédés décrits par V.Q. Yen et Coll. (J. Org. Chem., 23, 1858, 1958) et C.S. MARVEL et Coll. (Org.Synth. Coll., vol. I, 327) : 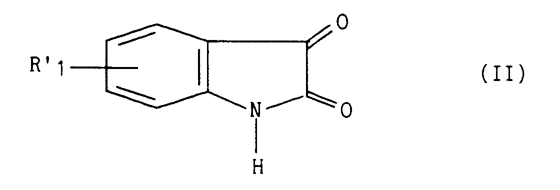 dans laquelle R'₁ représente un atome d'hydrogène, d'halogène, un groupement alkyle (C₁-C₆) linéaire ou ramifié, alkoxy (C₁-C₆) linéaire ou ramifié,
que l'on transforme en anion sodique correspondant en présence d'hydrure de sodium puis que l'on condense sur le magnésien de formule (III) : 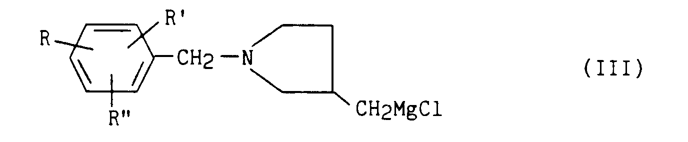 dans laquelle R, R', R'', identiques ou différents, représentent un atome d'halogène ou un groupement alkyle, alkoxy ou trihalogénométhyle, pour conduire au composé de formule (IV) : 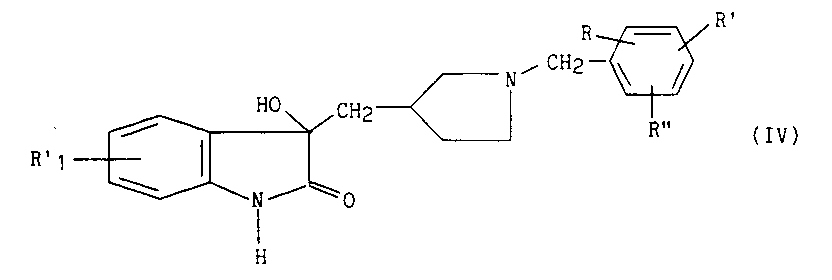 dans laquelle R'₁, R, R' et R'' ont la même signification que précédemment, qui subit l'action de l'hydrure de lithium aluminium pour conduire au composé de formule (I/a) : 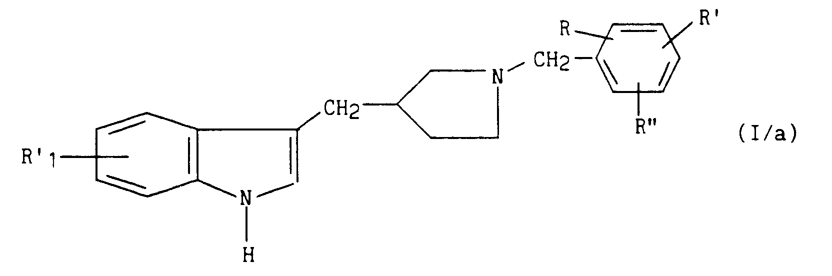 dans laquelle R'₁, R, R' et R'' ont la même signification que précédemment,
composé de formule (I/a) dont on transforme, si on le souhaite, le radical R'₁ lorsqu'il représente un atome de brome, en groupement cyano puis en l'un des autres groupements tels que définis dans la formule (I), selon des techniques classiques de la chimie organique, et qui subit, éventuellement,
l'action de l'acide chlorhydrique en solution éthanolique suivie d'une débenzylation par hydrogénolyse pour conduire au composé de formule (I/b), cas particulier des composés de formule (I) : 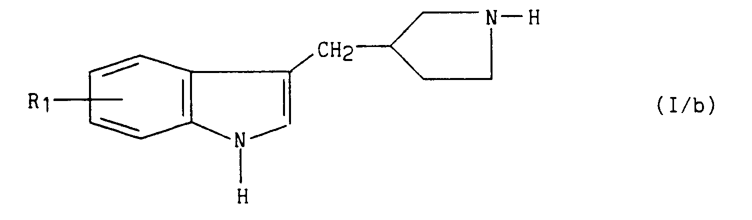 dans laquelle R₁ a la même signification que dans la formule (I),
puis, si on le souhaite, l'action du dérivé halogéné de formule (V) :

BrR'₃ (V)

dans laquelle R'₃ représente un groupement alkyle (C₁-C₆) linéaire ou ramifié éventuellement substitué par un groupement hydroxy,
pour conduire au composé de formule (I/c), cas particulier des composés de formule (I) : 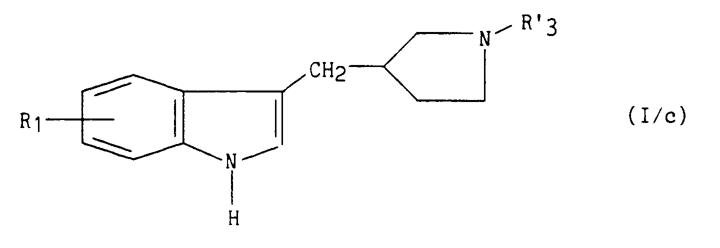 dans laquelle R₁ et R'₃ ont la même signification que précédemment,
composé de formule (I/a), (I/b) ou (I/c) que l'on soumet, le cas échéant, à l'action d'un dérivé iodé du benzène, substitué ou non, en présence de cuivre, d'un halogénure d'alkyle ou d'un halogénure d'acyle,
pour conduire au composé de formule (I/d), cas particulier des composés de formule (I) : 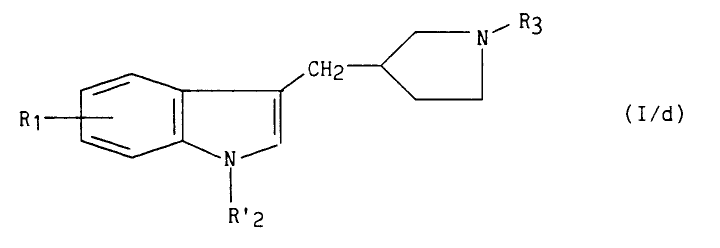 dans laquelle R₁ et R₃ ont la même signification que dans la formule (I) et R'₂ représente un groupement alkyle (C₁-C₆) linéaire ou ramifié, phényle (substitué ou non par un ou plusieurs atomes d'halogène ou groupements alkyle, alkoxy ou trihalogénométhyle) ou acyle,
composé de formule (I/a), (I/b), (I/c) ou (I/d), - que l'on purifie, le cas échéant, selon une technique classique de purification,

- dont on sépare, si on le souhaite, les isomères selon une technique classique de purification,

- et que l'on transforme, éventuellement, en ses sels d'addition à un acide pharmaceutiquement acceptable.

Les composés de formule (I) tels que R₁ représente un groupement : 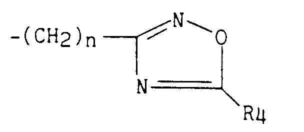 peuvent être préparés à partir d'un composé de formule (I₁), cas particulier des composés de formule (I) : 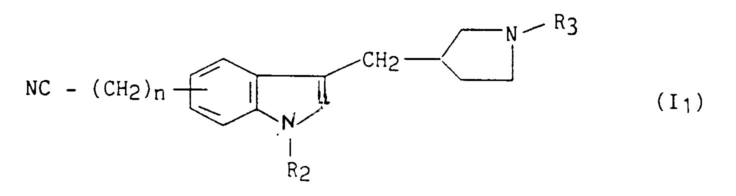 dans laquelle n, R₂ et R₃ ont la même signification que dans la formule (I),
que l'on fait réagir dans un premier temps avec de l'hydroxylamine puis dans un second temps avec un anhydride d'acide ou un ester, pour conduire au composé de formule (I₂), cas particulier des composés de formule (I) : 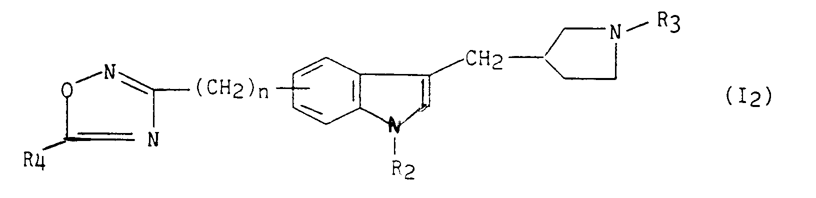 dans laquelle R₂, R₃, R₄ et n sont tels que définis dans la formule (I).

Les composés de formule (I) tels que R₁ représente un groupement : 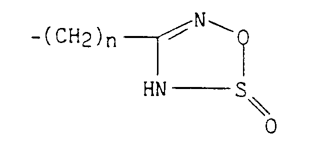 peuvent être préparés à partir d'un composé de formule (I₁) tel que défini précédemment, que l'on fait dans un premier temps réagir avec de l'hydroxylamine, puis dans un second temps avec du chlorure de thionyle, pour conduire au composé de formule (I₃), cas particulier des composés de formule (I) : 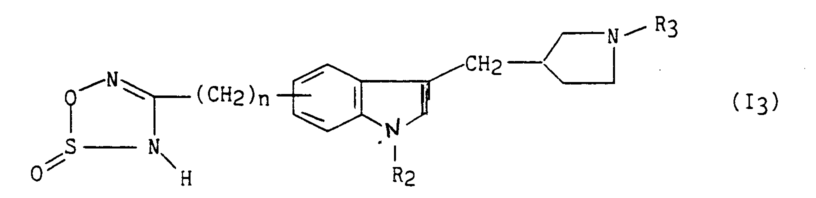 dans laquelle R₂, R₃ et n ont la même signification que précédemment.

Les composés de formule (I) tels que R₁ représente un groupement : 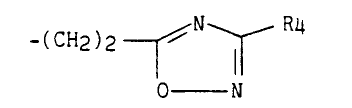 peuvent être préparés à partir d'un composé de formule (I₁) tel que défini précédemment dont on transforme le groupement nitrile en groupement acide, puis ester, qui subit alors l'action de l'oxime R₄C(NH₂) = NOH en présence d'hydrure de sodium pour conduire au composé de formule (I₄), cas particulier des composés de formule (I) : 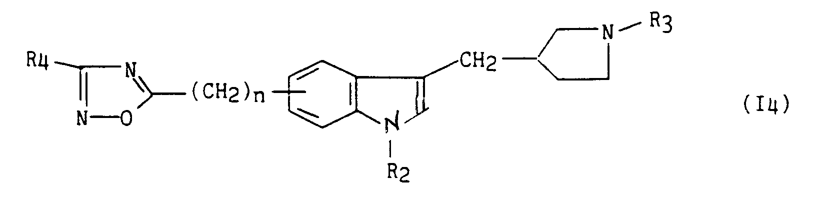 dans laquelle R₂, R₃, R₄, n, X et Y ont la même signification que dans la formule (I).

Les composés de la présente invention sont de puissants agonistes aux récepteurs 5-HT₁₋ₗᵢₖₑ et peuvent être utilisés dans le traitement de la dépression, de l'anxiété, de la migraine, de la douleur et des maladies associées à une déficience de la neurotransmission sérotoninergique comme il a été montré P.P.A. HUMPHREY et coll. (5-Hydroxytryptamine Mechanisms in Primary Headaches, p. 213-219, edited by J. OLESEN et P.R. SAXENA, Raven Press, N.Y., 1992).

Les composés de l'invention ont été évalués en tant qu'antimigraineux en comparaison avec un composé de référence, le sumatriptan, dans un test pharmacologique mesurant la contraction de la veine saphène isolée du chien ou du lapin comme décrit par P. HUHPHREY et coll. (Br. J. Pharmacol., 94, 1128, 1988).

L'invention s'étend aussi aux compositions pharmaceutiques renfermant comme principe actif au moins un composé de formule (I) avec un ou plusieurs excipients inertes, non toxiques et appropriés. Les compositions pharmaceutiques ainsi obtenues pourront être présentées sous diverses formes, les plus avantageuses étant les comprimés, les dragées, les gélules, suppositoires, suspensions buvables.

La posologie utile est adaptable selon la nature et la sévérité de l'affection, la voie d'administration ainsi que selon l'âge et le poids du patient. Cette posologie unitaire varie de 0,1 à 100 mg par jour en une ou plusieurs prises.

Les exemples suivants illustrent l'invention.

Les produits de départ utilisés sont des produits de départ connus ou préparés selon des modes opératoires connus.

### EXEMPLE 1 : 3-[(1-Benzylpyrrolidin-3-yl)méthyl]5-fluoroindole

### Stade A :3-Hydroxy-3[(1-benzylpyrrolidin-3-yl)méthyl]-5-fluoro-2-oxoindole

Préparer d'une part une solution de magnésien à partir de 334 mmoles de 1-benzyl-3-chlorométhylpyrrolidine, de 334 mmoles de magnésium et de 300 ml de tétrahydrofurane (THF). D'autre part, préparer une solution à partir de 303 mmoles de 5-fluoroisatine, 303 mmoles d'hydrure de sodium dans 500 ml de THF. Additionner à 0°C la solution de magnésien à cette deuxième solution contenant l'anion sodique de l'isatine. L'ensemble est laissé 3 heures à température ambiante puis porté 12 heures à reflux. Le mélange réactionnel est alors hydrolysé à froid sur 1 kg de glace, 500 ml d'eau de 60 ml d'acide acétique glacial. Le produit attendu est alors extrait au dichlorométhane et obtenu, sous forme d'huile, après séchage, évaporation et purification par chromatographie sur colonne de silice en utilisant comme éluant un mélange dichlorométhane/méthanol/ammoniaque (98/2/0,2).
Rendement : 65 %
Spectre infrarouge (nujol) :
v_{OH/NH} : entre 3500 et 2400 cm⁻¹
v_{CO} : 1712 cm⁻¹

### Stade B : 3-[(1-Benzylpyrrolidin-3-yl)méthyl]5-fluoroindole

Une solution contenant 33,7 mmoles du produit obtenu au stade précédent dans 300 ml de THF à 20°C est additionnée à une suspension contenant 33,7 mmoles d'hydrure de lithium-aluminium dans 200 ml de THF. Après 2 heures à température ambiante, l'ensemble est porté 2 heures à reflux puis est hydrolysé avec 20 ml d'eau, 28,5 ml de soude à 10 % et 55 ml d'eau. Après extraction au dichlorométhane, séchage et évaporation, le produit attendu est obtenu sous forme d'huile après purification par chromatographie sur colonne de silice en utilisant comme éluant un mélange dichlorométhane/méthanol/ammoniaque (98/2/0,2).
Rendement : 75 %
Spectre infrarouge (nujol) :
v_{NH} 3263 cm⁻¹

### EXEMPLE 2 : 3-[(Pyrrolidin-3-yl)méthyl]-5-fluoroindole, oxalate

24,5 mmoles du composé obtenu dans l'exemple 1 dans 150 ml d'éthanol et un équivalent d'acide chlorhydrique gazeux sont débenzylées, sous atmosphère d'hydrogène, à 20°C, en utilisant 0,8 g de palladium comme catalyseur. Après filtration de la solution et concentration, le produit attendu est salifié par un équivalent d'acide oxalique dans de l'éthanol.
Rendement : 95 %
Point de fusion : 180°C

| Microanalyse élémentaire | | | |
|---|---|---|---|
| | C % | H % | N % |
| calculé | 58,44 | 5,56 | 9,09 |
| trouvé | 59,15 | 5,68 | 8,91 |

### EXEMPLE 3 : 3-[(1-Propylpyrrolidin-3-yl)méthyl]-5-fluoroindole, oxalate

9 mmoles du produit obtenu dans l'exemple 2, 9 mmoles de 1-bromopropane et 20 mmoles de carbonate de sodium sont portées à 60°C pendant 8 heures dans 50 ml d'acétonitrile.
Après évaporation, reprise par de l'eau, extraction au dichlorométhane, séchage et évaporation, le produit attendu est obtenu sous forme d'huile et est purifié par chromatographie sur colonne de silice en utilisant comme éluant un mélange dichlorométhane/méthanol/ammoniaque (96/4/0,4). Il est transformé en oxalate dans de l'acide oxalique.
Point de fusion : 80°C

| Microanalyse élémentaire | | | |
|---|---|---|---|
| | C % | H % | N % |
| calculé | 61,70 | 6,62 | 7,99 |
| trouvé | 61,95 | 6,55 | 7,85 |

### EXEMPLE 4 : 3-[(1-Benzylpyrrolidin-3-yl)méthyl]-5-méthoxyindole, chlorhydrate

Ce composé a été synthétisé selon le même mode opératoire que celui décrit pour l'exemple 1 et a été transformé en chlorhydrate correspondant.
Point de fusion : 115°C

| Microanalyse élémentaire | | | | |
|---|---|---|---|---|
| | C % | H % | N % | Cl % |
| calculé | 70,67 | 7,06 | 7,85 | 9,93 |
| trouvé | 70,55 | 7,06 | 7,68 | 10,31 |

### EXEMPLE 5 : 3-[(Pyrrolidin-3-yl)méthyl]-5-méthoxyindole, chlorhydrate

Ce composé a été synthétisé selon le procédé décrit pour l'exemple 2 en utilisant le composé de l'exemple 4.
Le spectre de résonance magnétique nucléaire du proton montre la disparition des signaux correspondant au groupement benzyle.

### EXEMPLE 6 : 3-[(1-Benzylpyrrolidin-3-yl)méthyl]-5-méthylindole, chlorhydrate

Ce composé a été synthétisé selon le même mode opératoire que celui décrit par l'exemple 1 et a été transformé en chlorhydrate correspondant.
Rendement : 90 %
Point de fusion : 95°C

| Microanalyse élémentaire | | | | |
|---|---|---|---|---|
| | C % | H % | N % | Cl % |
| calculé | 73,99 | 7,39 | 8,22 | 10,40 |
| trouvé | 73,73 | 7,33 | 8,19 | 10,16 |

### EXEMPLE 7 : 3-[(Pyrrolidin-3-yl)méthyl]-5-méthylindole

Ce composé a été synthétisé selon le même procédé que celui décrit pour l'exemple 2 en utilisant le composé de l'exemple 6.
Rendement : 80 %
Le spectre de résonance magnétique nucléaire du proton montre la disparition des signaux correspondant au groupement benzyle.

### EXEMPLE 8 : 3-[(1-Propylpyrrolidin-3-yl)méthyl]-5-méthylindole, chlorhydrate

Ce composé a été synthétisé selon le même procédé que celui décrit dans l'exemple 3 en utilisant le composé de l'exemple 7.
Rendement : 54 %

| Microanalyse élémentaire | | | | |
|---|---|---|---|---|
| | C % | H % | N % | Cl % |
| calculé | 69,72 | 8,60 | 9,57 | 12,11 |
| trouvé | 70,23 | 8,64 | 9,14 | 11,85 |

### EXEMPLE 9 : 3-[(1-Benzylpyrrolidin-3-yl)méthyl]-5-chloroindole

Ce composé a été synthétisé selon le même procédé que celui décrit pour l'exemple 1.
Rendement : 85 %
Spectre infrarouge (nujol) :
v_{NH} : 3265 cm⁻¹

### EXEMPLE 10 : 3-[(Pyrrolidin-3-yl)méthyl]-5-chloroindole

Ce composé a été synthétisé selon le même procédé que celui décrit pour l'exemple 2 en utilisant le composé de l'exemple 9.
Le spectre de résonance magnétique nucléaire du proton montre la disparition des signaux correspondant au groupement benzyle.

### EXEMPLE 11 :1-(4-Fluorophényl)-3-[(1-benzylpyrrolidin-3-yl)méthyl]-5-chloroindole, oxalate

11 mmoles du composé de l'exemple 9, 16 mmoles de 1-fluoro-4-iodobenzène, 16 mmoles de carbonate de potassium, 1,5 mmoles de cuivre bronze et 6,6 mmoles de bromure de cuivre sont portées à 180°C dans 25 ml de N-méthylpyrrolidinone pendant 6 heures. L'ensemble est hydrolysé sur 150 ml d'acide chlorhydrique 1N et 20 ml d'éther isopropylique. Le chlorhydrate obtenu est filtré, repris à la soude et extrait au dichlorométhane. Après séchage et évaporation, le produit attendu est purifié par chromatographie sur colonne de silice en utilisant comme éluant un mélange dichlorométhane/méthanol (97/3). L'huile obtenue après concentration est alors salifiée par un équivalent d'acide oxalique dans l'éthanol.
Rendement : 70 %
Point de fusion : 196°C

| Microanalyse élémentaire | | | | |
|---|---|---|---|---|
| | C % | H % | N % | Cl % |
| calculé | 66,08 | 5,15 | 5,50 | 6,97 |
| trouvé | 66,22 | 5,24 | 5,43 | 7,18 |

### EXEMPLE 12 :3-[(1-Benzylpyrrolidin-3-yl)méthyl]-5-bromoindole, oxalate

Ce composé a été synthétisé en utilisant le même procédé que celui décrit dans l'exemple 1.
Rendement : 60 %

### EXEMPLE 13 : 3-[(Pyrrolidin-3-yl)méthyl]-5-bromoindole, oxalate

Ce composé a été synthétisé en utilisant le même procédé que celui décrit pour l'exemple 2 en utilisant le composé de l'exemple 12.

### EXEMPLE 14 : 3-[(1-Benzylpyrrolidin-3-yl)méthyl]-5-cyanoindole

13,5 mmoles du composé de l'exemple 12 et 16 mmoles de cyanure de cuivre sont portées au reflux de 50 ml de N-méthylpyrrolidone pendant 150 minutes.
L'ensemble est hydrolysé sur 50 ml de glace et 30 ml d'ammoniaque. Après extraction au dichlorométhane, séchage et évaporation, le produit attendu est purifié par chromatographie sur colonne de silice en utilisant comme éluant un mélange dichlorométhane/méthanol/ammoniaque (96/4/0,4).
Rendement : 55 %

### EXEMPLE 15 : 3-[(1-Benzylpyrrolidin-3-yl)méthyl]-5-aminocarbonylindole

0,47 mmoles du composé obtenu dans l'exemple 14 et 1 g d'acide polyphosphorique sont chauffées à 85°C pendant 90 minutes. L'ensemble est alors hydrolysé sur un mélange eau-glace et le pH de cette solution amené à 11-12 à l'aide de soude concentrée. Après extraction par un mélange chloroforme/méthanol (80/15), séchage et évaporation, le produit attendu est purifié par chromatographie sur colonne de silice en utilisant comme solvant un mélange THF/hexane/méthanol/ammoniaque (80/18/2/0,2).
Rendement : 60 %
Spectre infrarouge (nujol) :
v_{NH₂} : 3182 cm⁻¹
v_{CO} (amide) : 1651 cm⁻¹

### EXEMPLE 16 : 3-[(Pyrrolidin-3-yl)méthyl]-5-aminocarbonylindole

Ce composé est obtenu selon le même procédé que celui décrit pour l'exemple 2 en utilisant le composé de l'exemple 15.
Rendement : 86 %
Le spectre de résonance magnétique nucléaire du proton montre la disparition des signaux correspondant au groupement benzyle.

### EXEMPLE 17 : 3-[(1-Propylpyrrolidin-3-yl)méthyl]-5-aminocarbonylindole, oxalate

Ce composé est obtenu en utilisant le même procédé que celui décrit pour l'exemple 3 en utilisant le composé de l'exemple 16.
Rendement : 60 %

| Microanalyse élémentaire | | | |
|---|---|---|---|
| | C % | H % | N % |
| calculé | 60,79 | 6,71 | 11,19 |
| trouvé | 60,92 | 6,53 | 10,83 |

### EXEMPLE 20 : 3-[(1-Ethoxycarbonylpyrrolidin-3-yl)méthyl]-5-cyanoindole

Un mélange contenant 41 mmoles du composé de l'exemple 14 et 209 mmoles de chloroformiate d'éthyle dans 400 ml de toluène est porté à reflux deux heures. Après évaporation du solvant, le produit attendu est obtenu par purification du résidu par chromatographie sur colonne de silice en utilisant comme solvant un mélange dichlorométhane/méthanol/ammoniaque (98/2/0,2).
Rendement : 79 %
Spectre infrarouge (nujol) :
v_{NH} : 3255 cm-¹
v_{CO} : 1676 cm-¹

### EXEMPLE 21 : 3-[(1-Méthylpyrrolidin-3-yl)méthyl]-5-aminométhylindole

A 15,8 mmoles du composé de l'exemple 20 dans 50 ml de tétrahydrofurane est additionnée à 20°C une suspension contenant 158 mmoles d'hydrure de lithiumaluminium dans 15C ml de tétrahydrofurane. L'ensemble est maintenu 9 heures à 20°C puis hydrolysé par 30 ml d'eau et 12 ml de soude à 10 %. Après filtration et évaporation, le produit attendu est obtenu sous forme d'huile.
Rendement : 72 %

### EXEMPLE 22 : 3-[(1-Méthylpyrrolidin-3-yl)méthyl]-5-méthylsulfonamidométhylindole

A une solution contenant 10,6 mmoles du produit obtenu au stade précédent dans 80 ml de chloroforme sont additionnées, à 0°C, 10,6 mmoles de triéthylamine puis 10,6 mmoles du chlorure de l'acide méthane sulfonique. L'ensemble est abandonné deux heures à 20°C puis porté 3 heures à reflux. Après hydrolyse aqueuse, extraction au dichlorométhane, séchage et évaporation, le produit attendu est obtenu après purification par chromatographie sur colonne de silice en utilisant comme solvant un mélange dichlorométhane/méthanol/ammoniaque (80/20/2).
Rendement : 52 %
Spectre infrarouge :
v_{NH} : 3200 cm⁻¹
v_{SO₂} : 1311 et 1146 cm⁻¹

### EXEMPLE 23 : 1-(4-Fluorophényl)-3-[(1-éthoxycarbonylpyrrolidin-3-yl)méthyl]-5-chloroindole

Ce composé a été obtenu selon le même procédé que celui décrit dans l'exemple 20 en utilisant le composé de l'exemple 11.
Rendement : 70 %
Spectre infrarouge (nujol) :
v_{CO} : 1676 cm⁻¹

### EXEMPLE 24 : 1-(4-Fluorophényl)-3-[(pyrrolidin-3-yl)méthyl]-5-chloroindole, bromhydrate

0,64 mmoles du composé obtenu dans l'exemple 23 sont portées à reflux dans 1,4 ml d'acide bromhydrique à 48 % pendant 1 heure. Après refroidissement, le précipité est filtré, lavé à l'eau et à l'éther et conduit au produit attendu.
Rendement : 65 %
Point de fusion : 215°C

| Microanalyse élémentaire | | | | |
|---|---|---|---|---|
| | C % | H % | N % | Cl % |
| calculé | 55,70 | 4,67 | 6,84 | 8,65 |
| trouvé | 56,52 | 4,89 | 6,70 | 9,00 |

### Exemple 26 : 3-[(1-Benzylpyrrolidin-3-yl)méthyl]-5-aminométhylindole

Ce composé a été synthétisé selon le procédé décrit dans l'exemple 21 à partir du composé décrit dans l'exemple 14.
Rendement : 40 %

### Exemple 27 : 3-[(1-Benzylpyrrolidin-3-yl)méthyl]-5-méthylsulfonamidoindole, chlorhydrate

Ce composé a été synthétisé selon le procédé décrit dans l'exemple 22 à partir du composé décrit dans l'exemple 26.
Point de fusion : 90°C

| Microanalyse élémentaire | | | | | |
|---|---|---|---|---|---|
| | C % | H % | N % | Cl % | S % |
| calculé | 60,89 | 6,50 | 9,68 | 8,17 | 7,39 |
| trouvé | 60,61 | 6,74 | 9,49 | 8,09 | 7,73 |

### Exemple 28 : 1-Méthyl-3-[(1-benzylpyrrolidin-3-yl)méthyl]-5-bromoindole

Ce composé a été synthétisé selon le procédé décrit dans l'exemple 1 à partir du produit de départ correspondant.
Rendement : 45 %

### Exemple 29 : 1-Méthyl-3-[(1-benzylpyrrolidin-3-yl)-méthyl]-5-[2-(aminocarbonyl)vinyl]indole, chlorhydrate

Un mélange contenant 2,68 mmoles du composé de l'exemple 28, 3,53 mmoles d'acrylamide, 0,17 mmole de diacétate de palladium, 0,76 mmoles de triorthotolylphosphine, 0,4 ml de triéthylamine et 6 ml d'acétonitrile est chauffé à 100°C dans un réacteur hermétique pendant 24 heures. Le solvant est évaporé et le résidu purifié par chromatographie sur colonne de silice en utilisant comme éluant un mélange dichlorométhane/ méthanol/ammoniaque (95/5/0,5). Le produit attendu est alors obtenu par salification de l'huile dans une solution d'éthanol chlorhydrique et cristallisation dans de l'éther.
Spectre infrarouge (nujol) :
v_{CO} (amide) : 1664 cm⁻¹

### Exemple 30 : 1-Méthyl-3-[(1-benzylpyrrolidin-3-yl)méthyl]-5-[2-(diméthylaminocarbonyl)vinyl]indole, chlorhydrate

Le produit attendu est obtenu selon le procédé décrit dans l'exemple 29 en remplaçant l'acrylamide par le N,N-diméthylacrylamide.
Point de fusion : 55°C

| Microanalyse élémentaire | | | | |
|---|---|---|---|---|
| | C % | H % | N % | Cl % |
| calculé | 71,30 | 7,36 | 9,59 | 8,09 |
| trouvé | 71,03 | 7,09 | 9,42 | 8,02 |

### Exemple 31 : 1-Méthyl-3-[(1-benzylpyrrolidin-3-yl)méthyl]-5-[2-(aminocarbonyl)éth-1-yl]indole, chlorhydrate

8,64 mmoles du composé décrit dans l'exemple 29 dans 250 ml d'éthanol sont hydrogénées à température ambiante en présence de 400 mg de palladium sur charbon à 10 % comme catalyseur. Après filtration du catalyseur et évaporation du solvant, le résidu est répris par de l'éther et le produit attendu cristallise.
Point de fusion : 87°C
Spectre infrarouge (nujol) :
v_{CO} (amide) : 1660 cm⁻¹

### Exemple 32 : 1-Méthyl-3-[(1-benzylpyrrolidin-3-yl)méthyl]-5-[2-(aminosulfonyl)vinyl]indole

Le produit attendu est obtenu selon le procédé décrit dansl'exemple 29 en remplaçant l'acrylamide par la vinylsulfonamide.
Point de fusion : 120°C
Spectre infrarouge (nujol) :
v_{SO₂}: 1331 et 1146 cm⁻¹

### Exemple 33 : 1-Méthyl-3-[(1-benzylpyrrolidin-3-yl)méthyl]-5-[2-(aminosulfonyl)éthyl]indole, chlorhydrate

Le produit attendu est obtenu selon le procédé décrit dans l'exemple 31 à partir du composé décrit dans l'exemple 32.
Point de fusion : 88°C

| Microanalyse élémentaire | | | | |
|---|---|---|---|---|
| | C % | H % | N % | Cl % |
| trouvé | 61,66 | 6,75 | 9,38 | 7,91 |
| calculé | 61,54 | 6,88 | 9,52 | 7,76 |

### Exemple 34 : 1-Méthyl-3-[(1-benzylpyrrolidin-3-yl)méthyl]-5-cyanoindole

Le produit attendu est obtenu selon le procédé décrit dans l'exemple 14 en utilisant le composé de l'exemple 28.
Spectre infrarouge (film liquide) :
v_{C=N} : 2218 cm⁻¹

### Exemple 35 : 1-Méthyl-3-[(1-benzylpyrrolidin-3-yl)méthyl]-5-aminométhylindole

Le produit attendu est obtenu selon le procédé décrit dans l'exemple 21 en utilisant le composé de l'exemple 34.
Spectre infrarouge (nujol) :
v_{C=C} (aromatique) : 1603 cm⁻¹

### Exemple 36 : 1-Méthyl-3-[(1-benzylpyrrolidin-3-yl)méthyl]-5-(méthylsulfonylaminométhyl)indole, chlorhydrate

Le produit attendu est obtenu selon le procédé décrit dans l'exemple 22 en utilisant le composé de l'exemple 35.
Point de fusion : 140°C

| Microanalyse élémentaire | | | | | |
|---|---|---|---|---|---|
| | C % | H % | N % | S % | Cl % |
| calculé | 61,66 | 6,75 | 9,38 | 7,16 | 7,91 |
| trouvé | 61,63 | 6,86 | 9,39 | 7,16 | 8,01 |

### Exemple 37 : 1-Méthyl-3-[(1-éthoxycarbonylpyrrolidin-3-yl)méthyl]-5-cyanoindole

Le produit attendu est obtenu selon le procédé décrit dans l'exemple 20 en utilisant le composé de l'exemple 34.
Spectre infrarouge (nujol) :
v_{C=N} : 2218 cm⁻¹
v_{CO} : 1697 cm⁻¹

### Exemple 38 : 1-Méthyl-3-[(1-méthylpyrrolidin-3-yl)méthyl]-5-aminométhylindole

Le produit attendu est obtenu selon le procédé décrit dans l'exemple 21 en utilisant le composé de l'exemple 37.

### Exemple 39 : 1-Méthyl-3-[(1-méthylpyrrolidin-3-yl)méthyl]-5-(méthylsulfonylaminométhyl)indole, chlorhydrate

Le produit attendu est obtenu selon le procédé décrit dans l'exemple 22 en utilisant le composé de l'exemple 38.
Point de fusion : 84°C

### Exemple 40 : 1-Méthyl-3-[(1-benzylpyrrolidin-3-yl)méthyl]-5-[(2-oxoimidazolidino)méthyl]indole, chlorhydrate

A -5°C, 5,55 mmoles de β-chloroéthylisocyanate sont ajoutées à une solution contenant 5,55 mmoles du composé de l'exemple 35 dans 100 ml de chloroforme anhydre. Après évaporation du solvant à température ambiante, le résidu est repris par 100 ml de tétrahydrofuranne. 13 ml de butyllithium en solution à 15 % dans l'hexane sont ajoutés goutte à goutte à -5°C dans le mélange précédent. Après une heure de réaction à 0°C, le mélange est hydrolysé par une solution saturée de chlorure d'ammonium. Après décantation, extraction au dichlorométhane, séchage et évaporation, on obtient le produit attendu qui est purifié par chromatographie sur colonne de silice en utilisant comme éluant un mélange dichlorométhane/ méthanol/ammoniaque (95/5/0,5). Le chlorhydrate est obtenu par traitement dans de l'éthanol chlorhydrique.
Point de fusion : 130°C

### Exemple 41 : 3-[(1-Ethoxycarbonylpyrrolidin-3-yl)méthyl]-5-bromoindole

Le produit attendu est obtenu en utilisant le procédé décrit dans l'exemple 20 en utilisant le composé de l'exemple 12.

### Exemple 42 : 3-[(1-Méthylpyrrolidin-3-yl)méthyl]-5-bromoindole

Le produit attendu est obtenu selon le procédé décrit dans l'exemple 21 en utilisant le composé de l'exemple 41.

### Exemple 43 : 3-[(1-Méthylpyrrolidin-3-yl)méthyl]-5-[2-(méthylaminosulfonyl)vinyl]indole

Le produit attendu est obtenu selon le procédé décrit dans l'exemple 29 en utilisant le composé de l'exemple 42 et en remplaçant l'acrylamide par la N-méthylvinylsulfonamide (préparée selon le procédé décrit dans le brevet US 3,761,473).

### Exemple 44 : 3-[(1-Méthylpyrrolidin-3-yl)méthyl]-5-[2-(méthylaminosulfonyl)éthyl]indole, chlorhydrate

Le produit attendu est obtenu selon le procédé décrit dans l'exemple 31 en utilisant le composé de l'exemple 43.
Spectre de masse : Ionisation NH₃ :
M+H⁺ : m/z = 336 (M théorique : 335)

### Exemple 45 : 3-[(1-Benzylpyrrolidin-3-yl)méthyl]-5-[5-méthyl-1,2,4-oxadiazol-3-yl]indole

Une solution contenant 12,7 mmoles de méthylate de sodium dans 5 ml de méthanol est additionnée à une solution contenant 12,7 mmoles de chlorhydrate d'hydroxylamine dans 15 ml de méthanol. Le mélange est agité une heure à température amibante. Après filtration du précipité, 5,7 mmoles du composé de l'exemple 14 sont ajoutées au filtrat et l'ensemble est porté à reflux 48 heures. Après évaporation du solvant, le solide brun obtenu est mélangé à 10 ml d'anhydride acétique. L'ensemble est chauffé 24 heures à 80°C. Après hydrolyse avec de l'eau, l'ensemble est amené à pH = 12 à l'aide de soude 2N. Après extraction au dichlorométhane, séchage et évaporation, le produit attendu est obtenu après purification du résidu par chromatographie sur colonne de silice en utilisant comme éluant un mélange dichlorométhane/méthanol/ammoniaque (95/5/0,5).

### Exemple 46 : 3-[(1-Benzylpyrrolidin-3-yl)méthyl]-5-[1,2,3,5-oxathiadiazol-2-oxo-4-yl]indole

Le produit attendu est obtenu selon le procédé décrit dans l'exemple 45 en remplaçant l'anhydride acétique par le chlorure de thionyle.

### Exemple 47 : 3-[(1-Benzylpyrrolidin-3-yl)méthyl]-5-[3-méthyl-1,2,4-oxadiazol-5-yl]indole

### ETUDE PHARMACOLOGIQUE DES COMPOSES DE L'INVENTION

### EXEMPLE 48 : Contraction de la veine saphène

Les expériences sont effectuées sur des veines saphènes de chiens (10-25 kg) ou de lapins (2-3 kg) anesthésiés avec du pentobarbital (30 mg/kg i.v.). Les veines saphènes sont rapidement prélevées et découpées en anneaux. Ces anneaux sont montés entre deux crochets dans des cuves thermostatées à 37°C contenant de la solution physiologique (composition en mM : NaCl 118.3 ; KCl 4.7 ; CaCl₂ 2.5 ; MgSO₄ 1.2 ; KH₂PO₄ 1.2 ; NaHCO₃ 25.0 ; Ca-EDTA 0.026 et glucose 11.1).

La solution physiologique est bullée par un mélange de 95 % O₂ - 5 % CO₂. Le crochet inférieur consiste le point fixe tandis que le crochet supérieur est relié à un capteur de force isométrique. Les tissus sont mis sous une tension de base de 1.5 grammes (chien) et de 1 gramme (lapin). Les substances pharmacologiques étudiées sont préparées immédiatement avant l'usage ; elles sont solubilisées dans l'eau ou dans le diméthyl sulfoxyde.

Après le montage, les préparations sont laissées en repos pendant 60 minutes, des rinçages étant effectués toutes les 30 minutes. L'organe est alors mis en présence de phénoxybenzamine ( 5 x 10⁻⁸M) pendant 20 minutes. Cet agent est éliminé par plusieurs lavages successifs pendant 45 minutes. Après réajustement de la tension de base, une contraction est provoquée par le KCl (100 mM). Après lavage et retour à la ligne de base, une contraction est induite par la 5-hydroxytryptamine (10⁻⁵M).

Après lavage et retour à la ligne de base, une courbe dose-réponse aux substances pharmacologiques est effectuée par adjonction de doses cumulatives (10⁻⁹ à 10⁻⁴M).

Cette expérience permet de calculer la concentration efficace 50 % (EC₅₀) des composés de l'invention.

Cette EC₅₀ est calculée de la manière suivante : les valeurs de tension sont d'abord converties en pourcentages par rapport à l'effet maximum induit par le KCl. La concentration efficace 50 % (EC₅₀) est déterminée par régression non linéaire suivant le modèle de la loi d'action de masse de Michaelis-Menten.

La spécificité pour le récepteur 5-HT₁ de la veine est vérifiée en utilisant des antagonistes spécifiques tel que la métitépine (antagoniste 5-HT₁ / 5-HT₂) et la kétansérine (antagoniste 5-HT₂).

Dans ce test, l'EC₅₀ déterminée sur la veine saphène de chien pour le composé de l'exemple 17 est égale à 0,34 µM alors que celle du sumatriptan est égale à 0,64 µM.

Sur la veine saphène de lapin, l'EC₅₀ du composé de l'exemple 17 est égale à 0,41 µM.

### COMPOSITION PHARMACEUTIQUE

### EXEMPLE 49 : Composition pharmaceutique

| Formule de préparation pour 1000 comprimés dosés à 10 mg : | |
|---|---|
| Composé de l'exemple 17 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 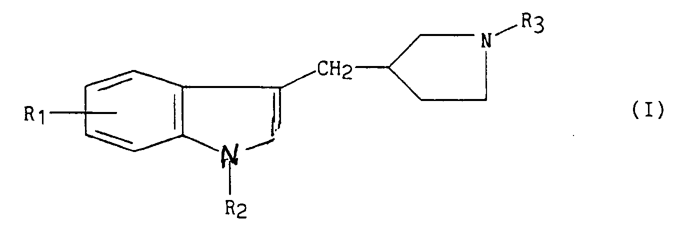 dans laquelle :
R₁ représente un atome d'hydrogène, d'halogène, un groupement alkyle (C₁-C₆) linéaire ou ramifié, alkoxy (C₁-C₆) linéaire ou ramifié. cyano, 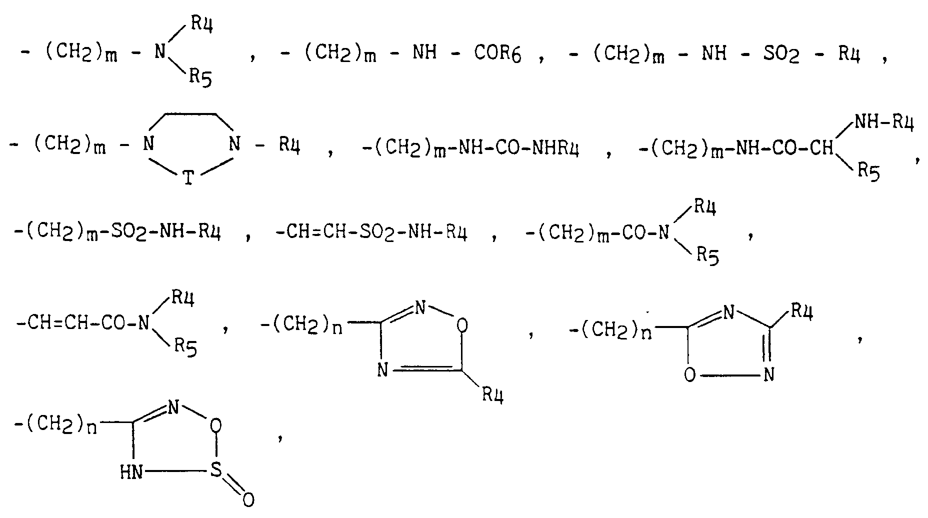 aminocarbonyle, ou l'un quelconque des groupements suivants : dans lesquels :
m est égal à 1, 2 ou 3,
n est égal à 0, 1 ou 2,
T représente un CO ou SO₂,
R₄ ou R₅, identiques ou différents, représentent un atome d'hydrogène, un groupement alkyle (C₁-C₆) linéaire ou ramifié ou phényle (substitué ou non par un ou plusieurs atomes d'halogène ou groupement trihalogénométhyle),
R₂ représente un atome d'hydrogène, un groupement alkyle (C₁-C₆) linéaire ou ramifié ou phényle (substitué ou non par un ou plusieurs atomes d'halogène ou groupements trihalogénométhyle),
R₃ représente un atome d'hydrogène, un groupement alkyle (C₁-C₆) linéaire ou ramifié (éventuellement substitué par un groupement hydroxy ou phényle (lui-même substitué ou non par un ou plusieurs atomes d'halogène ou groupements trihalogénométhyle)) ou alkoxycarbonyle (C₁-C₆) linéaire ou ramifié,
leurs énantiomères, diastéréoisomères et épimères ainsi que leurs sels d'addition à un acide pharmaceutiquement acceptable.

2. Composés de formule (I) selon la revendication 1 dans laquelle R₂ représente un atome d'hydrogène.

3. Composés de formule (I) selon la revendication 1 dans laquelle R₁ est situé en position 5 du noyau phényle.

4. Composés de formule (I) selon la revendication 1 dans laquelle R₃ représente un groupement benzyle.

5. Composés de formule (I) selon la revendication 1 dans laquelle R₃ représente un groupement alkyle (C₁-C₆) linéaire ou ramifié.

6. Composé de formule (I) selon la revendication 1 qui est le 3-[(1-propylpyrrolidin-3-yl)méthyl]-5-aminocarbonylindole.

7. Procédé de préparation des composés de formule (I) selon la revendication 1 caractérisé en ce que l'on utilise comme produit de départ une isatine de formule (II) : 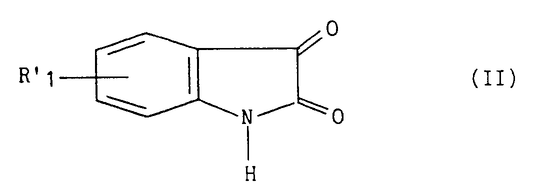 dans laquelle R'₁ représente un atome d'hydrogène, d'halogène, un groupement alkyle (C₁-C₆) linéaire ou ramifié, alkoxy (C₁-C₆) linéaire ou ramifié,
que l'on transforme en anion sodique correspondant en présence d'hydrure de sodium puis que l'on condense sur le magnésien de formule (III) : 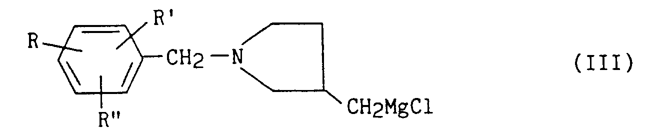 dans laquelle R, R', R'', identiques ou différents, représentent un atome d'halogène ou un groupement alkyle, alkoxy ou trihalogénométhyle,
pour conduire au composé de formule (IV) : 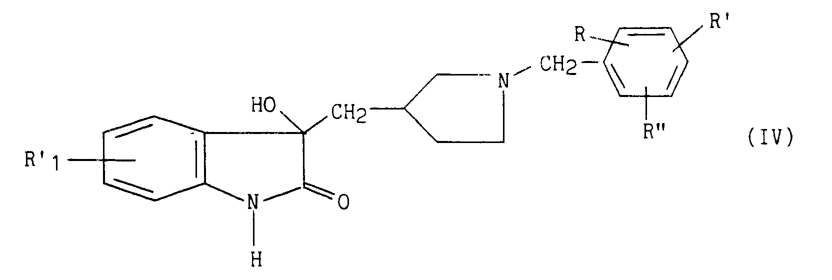 dans laquelle R'₁, R, R' et R'' ont la même signification que précédemment, qui subit l'action de l'hydrure de lithium aluminium pour conduire au composé de formule (I/a) : 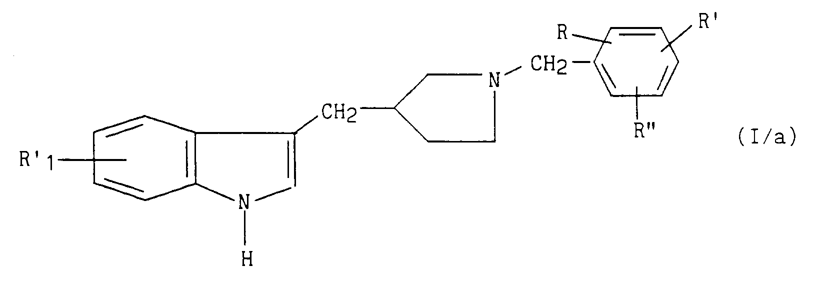 dans laquelle R'₁, R, R' et R'' ont la même signification que précédemment,
composé de formule (I/a) dont on transforme, si on le souhaite, le radical R'₁ lorsqu'il représente un atome de brome, en groupement cyano puis en l'un des autres groupements tels que définis dans la formule (I), selon des techniques classiques de la chimie organique, et qui subit, éventuellement,
l'action de l'acide chlorhydrique en solution éthanolique suivie d'une débenzylation par hydrogénolyse pour conduire au composé de formule (I/b), cas particulier des composés de formule (I) : 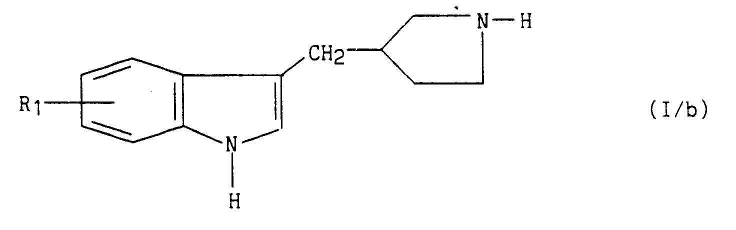 dans laquelle R₁ a la même signification que dans la formule (I),
puis, si on le souhaite, l'action du dérivé halogéné de formule (V) :
BrR'₃ (V)
dans laquelle R'₃ représente un groupement alkyle (C₁-C₆) linéaire ou ramifié éventuellement substitué par un groupement hydroxy,
pour conduire au composé de formule (I/c), cas particulier des composés de formule (I) : 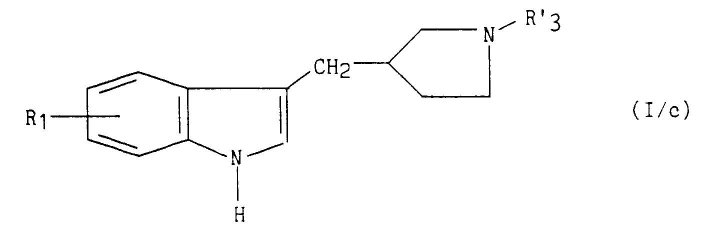 dans laquelle R₁ et R'₃ ont la même signification que précédemment,
composé de formule (I/a), (I/b) ou (I/c) que l'on soumet, le cas échéant, à l'action d'un dérivé iodé du benzène, substitué ou non, en présence de cuivre, d'un halogénure d'alkyle ou d'un halogénure d'acyle,
pour conduire au composé de formule (I/d), cas particulier des composés de formule (I) : 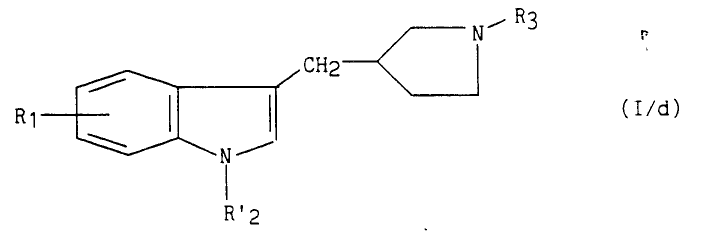 dans laquelle R₁ et R₃ ont la même signification que dans la formule (I) et R'₂ représente un groupement alkyle (C₁-C₆) linéaire ou ramifié, phényle (substitué ou non par un ou plusieurs atomes d'halogène ou groupements alkyle, alkoxy ou trihalogénométhyle) ou acyle,
composé de formule (I/a), (I/b), (I/c) ou (I/d),
- que l'on purifie, le cas échéant, selon une technique classique de purification,
- dont on sépare, si on le souhaite, les isomères selon une technique classique de purification,
- et que l'on transforme, éventuellement, en ses sels d'addition à un acide pharmaceutiquement acceptable.

8. Compositions pharmaceutiques contenant comme principe actif au moins un composé selon l'une quelconque revendications 1 à 6, seul ou en combinaison avec un ou plusieurs véhicules inertes, non toxiques pharmaceutiquement acceptables.

9. Compositions pharmaceutiques selon la revendication 8 utiles dans le traitement de la migraine et des maladies associées.

## Claims

1. Compounds of formula (I): 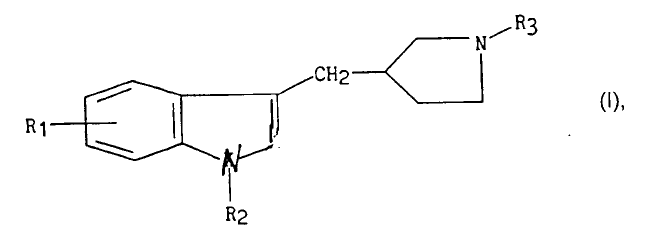 wherein:
R₁ represents a hydrogen or halogen atom, a linear or branched (C₁-C₆)alkyl, linear or branched (C₁-C₆) alkoxy, cyano or aminocarbonyl group, or any one of the 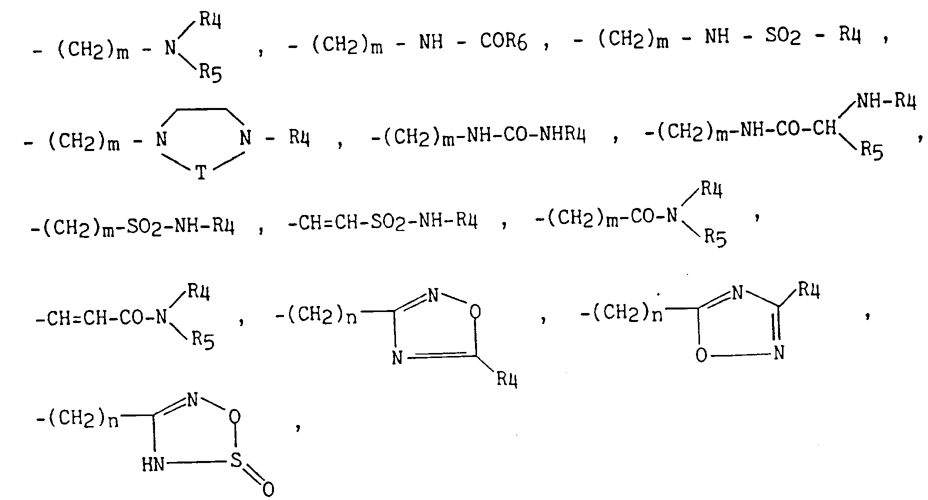 following groups: wherein:
m is 1,2 or 3,
n is 0,1 or 2,
T represents a CO or SO₂ group,
R₄ and R₅, which may be identical or different, represent a hydrogen atom, a linear or branched (C₁-C₆)alkyl or phenyl group (unsubstituted or substituted by one or more halogen atoms or trihalomethyl groups),
R₂ represents a hydrogen atom, a linear or branched (C₁-C₆)alkyl or phenyl group (unsubstituted or substituted by one or more halogen atoms or trihalomethyl groups),
R₃ represents a hydrogen atom, a linear or branched (C₁-C₆)alkyl group (optionally substituted by a hydroxy group or a phenyl group (itself unsubstituted or substituted by one or more halogen atoms or trihalomethyl groups)) or a linear or branched (C₁-C₆)alkoxycarbonyl group,
enantiomers, diastereoisomers and epimers thereof and addition salts thereof with a pharmaceutically acceptable acid.

2. Compounds of formula (1) according to claim 1, wherein R₂ represents a hydrogen atom.

3. Compounds of formula (I) according to claim 1, wherein R₁ is located in the 5-position of the phenyl nucleus.

4. Compounds of formula (I) according to claim 1, wherein R₃ represents a benzyl group.

5. Compounds of formula (I) according to claim 1, wherein R₃ represents a linear or branched (C₁-C₆)alkyl group.

6. Compound of formula (I) according to claim 1 that is 3-[(1-propylpyrrolidin-3-yl)methyl]-5-aminocarbonylindole.

7. Process for the preparation of compounds of formula (I) according to claim 1, characterised in that there is used as starting material an isatin of formula (II): 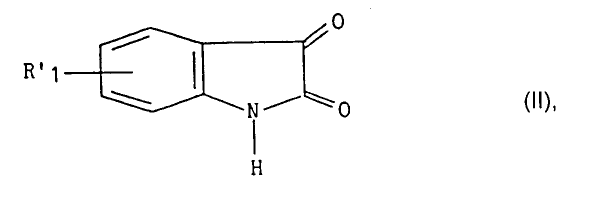 wherein R'₁ represents a hydrogen atom, a halogen atom, a linear or branched (C₁-C₆)alkyl group or a linear or branched (C₁-C₆)alkoxy group,
which is converted into the corresponding anion with sodium in the presence of sodium hydride, which is then condensed with the magnesium compound of formula (III): 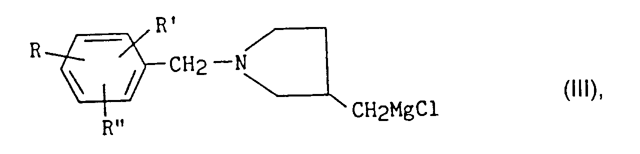 wherein R, R', R'', which may be identical or different, represent a halogen atom or an alkyl, alkoxy or trihalomethyl group,
to yield a compound of formula (IV): 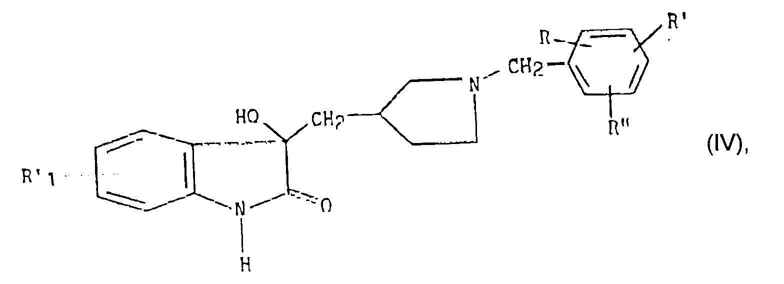 wherein R'₁, R, R' and R'' are as defined hereinbefore,
which is subjected to the action of lithium aluminium hydride to yield a compound of formula 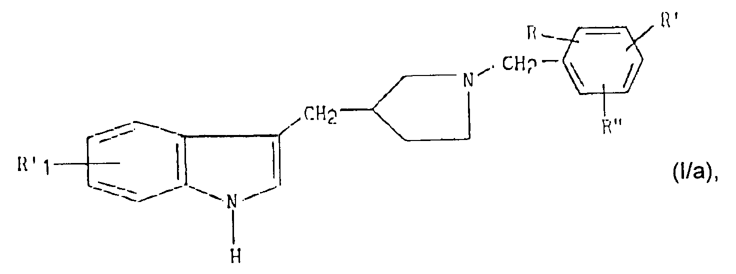 (I/a): wherein R'₁, R, R' and R'' are as defined hereinbefore,
the R'₁ radical of which compound of formula (I/a), when that radical represents a bromine atom, is, if desired, converted into a cyano group and then into one of the other groups as defined for formula (I), according to conventional techniques of organic chemistry, and which compound is optionally subjected
to the action of ethanolic HCl solution followed by debenzylation by hydrogenolysis to yield a compound of formula (I/b), a particular case of the compounds of formula (I): 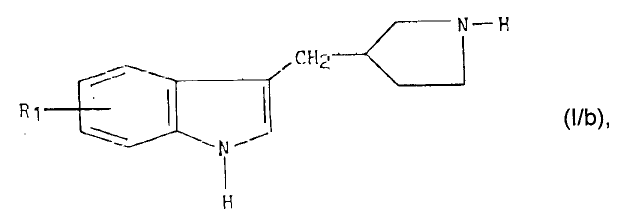 wherein R₁ is as defined for formula (I),
and then, if desired, to the action of the halogen compound of formula (V):
BrR'₃ (V)
, wherein R'₃ represents a linear or branched (C₁-C₆)alkyl group optionally substituted by a hydroxy group,
to yield a compound of formula (I/c), a particular case of the compounds of formula (I): 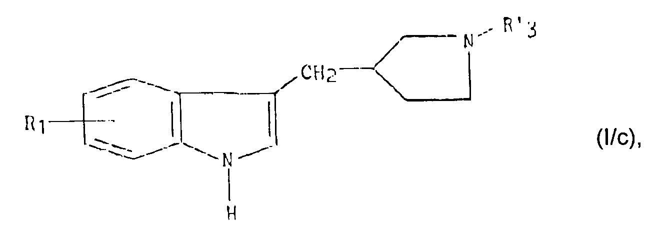 wherein R₁ and R'₃ are as defined hereinbefore,
which compound of formula (I/a), (I/b) or (I/c) is subjected, where necessary, to the action of an iodinated benzene compound, which may be unsubstituted or substituted, in the presence of copper, or to the action of an alkyl halide or of an acyl halide,
to yield a compound of formula (I/d), a particular case of the compounds of formula (I): 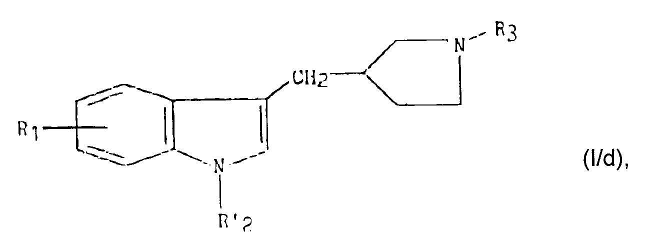 wherein R₁ and R₃ are as defined for formula (I) and R'₂ represents a linear or branched (C₁-C₆)alkyl, phenyl (unsubstituted or substituted by one or more halogen atoms or alkyl, alkoxy or trihalomethyl groups) or acyl group,
which compound of formula (I/a), (I/b), (I/c) or (I/d)
- is purified, if necessary, according to a conventional purification technique,
- is separated, if desired, into its isomers according to a conventional purification technique,
- is converted, where appropriate, into its addition salts with a pharmaceutically acceptable acid.

8. Pharmaceutical compositions comprising as active ingredient at least one compound according to any one of claims 1 to 6, alone or in combination with one or more pharmaceutically acceptable, inert, non-toxic carriers.

9. Pharmaceutical compositions according to claim 8 for use in the treatment of migraine and associated disorders.

## Patentansprüche

1. Verbindungen der Formel (I): 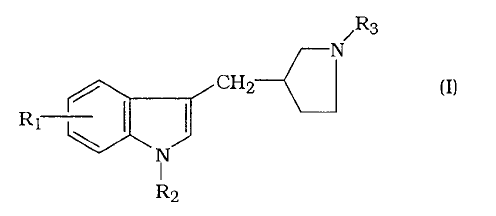 in der:
R₁ ein Wasserstoffatom, ein Halogenatom, eine geradkettige oder verzweigte (C₁-C₆)-Alkylgruppe, eine geradkettige oder verzweigte (C₁-C₆)-Alkoxygruppe, eine Cyanogruppe, eine Aminocarbonylgruppe oder eine der folgenden Gruppen: 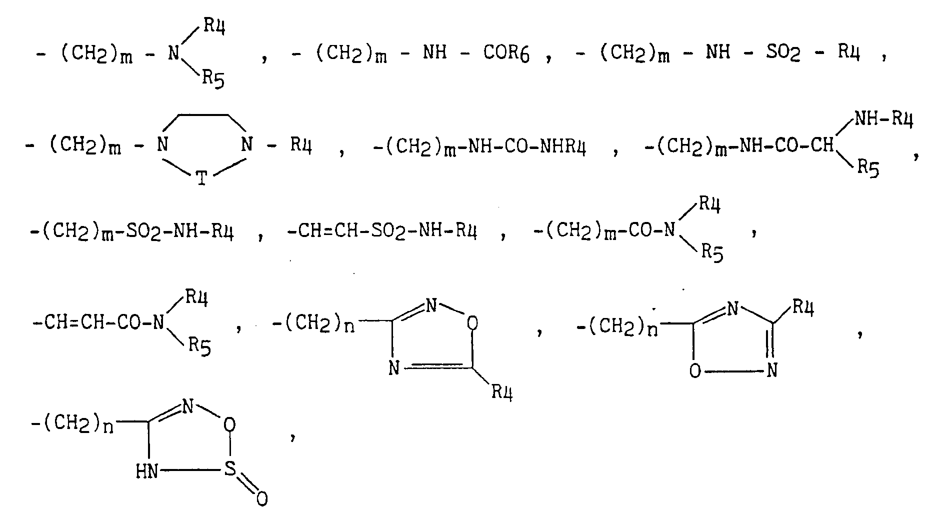 worin:
m 1, 2 oder 3,
n 0, 1 oder 2,
T CO oder SO₂,
R₄ und R₅, die gleichartig oder verschieden sind, ein Wasserstoffatom, eine geradkettige oder verzweigte (C₁-C₆)-Alkylgruppe oder eine (gegebenenfalls durch eines oder mehrere Halogenatome oder Trihalogenmethylgruppen substituierte) Phenylgruppe darstellen,
R₂ ein Wasserstoffatom, eine geradkettige oder verzweigte (C₁-C₆)-Alkylgruppe oder eine (gegebenenfalls durch eines oder mehrere Halogenatome oder Trihalogenmethylgruppen substituierte) Phenylgruppe und
R₃ ein Wasserstoffatom, eine geradkettige oder verzweigte (gegebenenfalls durch eine Hydroxygruppe oder Phenylgruppe (die ihrerseits gegebenenfalls durch eines oder mehrere Halogenatome oder Trihalogenmethylgruppen substituiert sein kann) substituierte) (C₁-C₆)-Alkylgruppe oder eine geradkettige oder verzweigte (C₁-C₆)-Alkoxycarbonylgruppe bedeuten,
deren Enantiomere, Diastereoisomere und Epimere sowie deren Additionssalze mit einer pharmazeutisch annehmbaren Säure.

2. Verbindungen der Formel (I) nach Anspruch 1, worin R₂ ein Wasserstoffatom bedeutet.

3. Verbindungen der Formel (I) nach Anspruch 1, worin R₁ in der 5-Stellung des Phenylkerns gebunden ist.

4. Verbindungen der Formel (I) nach Anspruch 1, worin R₃ eine Benzylgruppe darstellt.

5. Verbindungen der Formel (I) nach Anspruch 1, worin R₃ eine geradkettige oder verzweigte (C₁-C₆)-Alkylgruppe darstellt.

6. Verbindung der Formel (I) nach Anspruch 1, nämlich 3-[(1-Propylpyrrolidin-3-yl)-methyll-5-aminocarbonylindol.

7. Verfahren zur Verbindungen der Formel (I) nach Anspruch 1, dadurch gekennzeichnet, daß man als Ausgangsprodukt ein Isatin der Formel (II) verwendet: 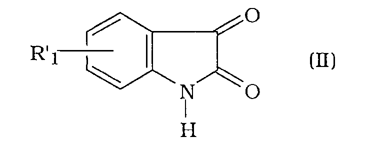 in der R'₁ ein Wasserstoffatom, ein Halogenatom, eine geradkettige oder verzweigte (C₁-C₆)-Alkylgruppe oder eine geradkettige oder verzweigte (C₁-C₆)-Alkylgruppe darstellt,
welches man in Gegenwart von Natriumhydrid in das entsprechende Natriumanion umwandelt und dann mit der Magnesiumverbindung der Formel (III) kondensiert: 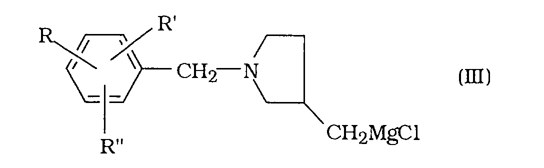 in der R, R', R'', die gleichartig oder verschieden sind, Halogenatome, Alkylgruppen, Alkoxygruppen oder Trihalogenmethylgruppen bedeuten,
zur Bildung der Verbindung der Formel (IV): 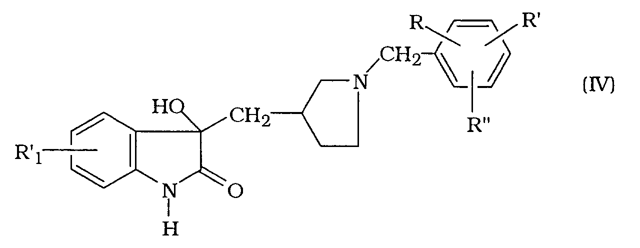 in der R'₁, R, R' und R'' die oben angegebenen Bedeutungen besitzen, welche man der Einwirkung von Lithiumaluminiumhydrid unterwirft zur Bildung der Verbindung der Formel (I/a): 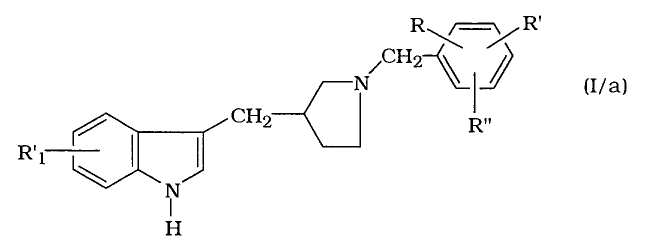 in der R'₁, R, R' und R'' die oben angegebenen Bedeutungen besitzen,
in welcher Verbindung der Formel (I/a) man gewünschtenfalls die Gruppe R'₁, wenn sie ein Bromatom darstellt, in eine Cyanogruppe und dann in eine der anderen Gruppen, wie sie bezüglich der Formel (I) angegeben sind, mit Hilfe klassischer Methoden der organischen Chemie umwandelt und gegebenenfalls
der Einwirkung von Chlorwasserstoffsäure in ethanolischer Lösung gefolgt von einer Debenzylierung durch Hydrogenolyse unterwirft zur Bildung der Verbindung der Formel (I/b), einem Sonderfall der Verbindungen der Formel (I): 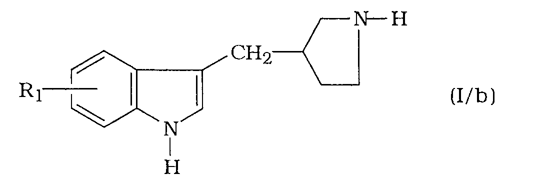 in der R₁ die bezüglich der Formel (I) angegebenen Bedeutungen besitzt, welche man gewünschtenfalls der Einwirkung eines Halogenderivats der Formel (V):
BrR'₃ (V)
unterwirft, in der R'₃ eine geradkettige oder verzweigte, gegebenenfalls durch eine Hydroxygruppe substituierte (C₁-C₆)-Alkylgruppe darstellt, so daß man die Verbindung der Formel (I/c) erhält, einem Sonderfall der Verbindungen der Formel (I): 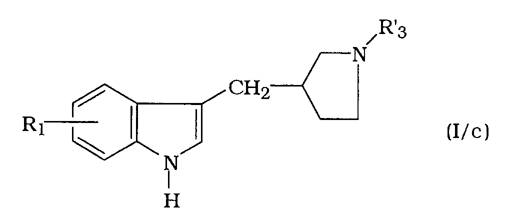 in der R₁ und R'₃ die oben angegebenen Bedeutungen besitzen,
welche Verbindung der Formeln (I/a), (I/b) oder (I/ c) man gegebenenfalls der Einwirkung eines gegebenenfalls substituierten Benzoliodderivats in Gegenwart von Kupfer, eines Alkylhalogenids oder eines Acylhalogenids unterwirft, zur Bildung der Verbindung der Formel (I/d), einem Sonderfall der Verbindungen der Formel (I): 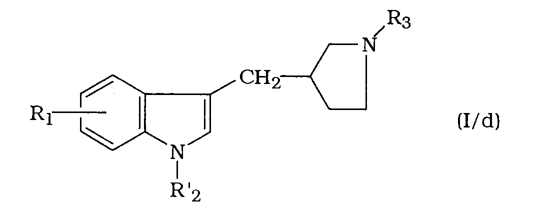 in der R₁ und R₃ die bezüglich der Formel (I) angegebenen Bedeutungen besitzen und R'₂ eine geradkettige oder verzweigte (C₁-C₆)-Alkylgruppe, eine (gegebenenfalls durch ein oder mehrere Halogenatome oder Alkyl-, Alkoxy oder Trihalogenmethylgruppen substituierte) Phenylgruppe oder eine Acylgruppe bedeutet,
welche Verbindungen der Formeln (I/a), (I/b), (I/c) und (I/d) man
- gegebenenfalls mit Hilfe einer klassischen Reinigungsmethode reinigt,
- gewünschtenfalls mit Hilfe einer klassischen Reinigungstechnik in die Isomeren auftrennt und
- gegebenenfalls in ihre Additionssalze mit einer pharmazeutisch annehmbaren Säure umwandelt.

8. Pharmazeutische Zubereitungen enthaltend als Wirkstoff mindestens eine Verbindung nach einem der Ansprüche 1 bis 6 allein oder in Kombination mit einem oder mehreren inerten, nichttoxischen, pharmazeutisch annehmbaren Trägermaterialien.

9. Pharmazeutische Zubereitungen nach Anspruch 8 zur Behandlung der Migräne und damit verknüpften Erkrankungen.
